(19) Europäisches Patentamt
European Patent Office
Office européen des brevets

(11) Publication number: 0 115 681
A1

# (12) EUROPEAN PATENT APPLICATION

(21) Application number: 83307533.6

(22) Date of filing: 12.12.83

(51) Int. Cl.³: G 01 N 33/54, G 01 N 33/56

(30) Priority: 03.01.83 US 454928

(43) Date of publication of application: 15.08.84
Bulletin 84/33

(84) Designated Contracting States: AT BE CH DE FR GB LI NL SE

(71) Applicant: WARNER-LAMBERT COMPANY, 201 Tabor Road, Morris Plains New Jersey 07950 (US)

(72) Inventor: Barnes, Wayne Riley, 14534 Sunrose, Farmes Branch Texas, 75234 (US)
Inventor: Johnson, Teresa, 3803 High Grove, Dallas Texas, 75220 (US)

(74) Representative: Jones, Michael Raymond et al, HASELTINE LAKE & CO. 28 Southampton Buildings Chancery Lane, London WC2A 1AT (GB)

## (54) Process for producing a solid phase immunoassay.

(57) There is disclosed a process for preparing a solid phase immunoassay, which process comprises:

purifying a specific binding protein;

reacting the purified specific binding protein with one or more aldehyde to enhance the hydrophobic nature of the protein without crosslinking or polymerizing the protein;

contacting the surface of an insoluble support with the enhanced hydrophobic specific binding protein in the presence of a buffer and bonding the specific binding protein to the surface; and

removing any remaining unbound protein from the insoluble support.

An associated method and kit for assaying a specimen for a specific component of the specimen are also disclosed. The invention enables the protein to be bound to the insoluble support more securely than in the absence of the aldehyde.

EP 0 115 681 A1

# PROCESS FOR PRODUCING A SOLID PHASE IMMUNOASSAY

The present invention relates to a process for preparing a solid phase immunoassay and to a method of performing an immunoassay procedure and kits therefor.

Many methods of preparing solid phase immunoassays have been proposed. These methods have employed both active and passive adsorption techniques. One of the passive techniques is disclosed in U.S. Patent No. 3646346 wherein a water-insoluble material, such as the inner surface of a test tube, is contacted with a protein and the surface becomes coated with the protein by passive adsorption. The coated tube is then brought into contact with an aqueous sample containing the material to be assayed in the presence of a known quantity of labelled material. While this process was originally successful, a disadvantage typically found with passively coated protein is the susceptibility to desorption during the assay incubation which results in inconsistent results.

Attempts have been made to overcome the difficulties associated with passive adsorption by employing active adsorption techniques. These latter techniques have primarily used covalent bonding agents to couple the protein to the solid support. One such technique is disclosed in U.S. Patent No. 4001583 wherein biological substances are covalently bonded to the inner surface of a plastic vessel which inner surface has been previously coated with glutaraldehyde, with or without prior treatment with an aliphatic amine or diamine. It is asserted that the coupling effect is achieved by the self-polymerization of the aldehyde material on the surface of the plastic followed by a Schiff base-type coupling of the protein to the active unreacted aldehyde groups.

In U.S. Patent No. 4069352, another process is described for performing an active adsorption technique. This patent discloses a solid phase immunoadsorbent prepared by crosslinking a low titre antiserum to form a protein structure having an increased molecular weight. In general the extent of the crosslinking desired for optimum results is in the range from two to five times the molecular weight of the uncrosslinked antibody or binding protein starting material. This crosslinked low titre material is then adsorbed onto the surface of a water insoluble polymeric material, which is, for example, in the form of a test tube, to form the solid phase immunoadsorbent. The adsorption reaction is performed at a pH in the range from 8 to 10. The crosslinking procedure may be performed using a bifunctional reagent, such as glutaraldehyde, before bringing the crosslinked antiserum into contact with the surface of the polymeric material at the

critical pH.

According to the first aspect of the present invention, there is provided a process for preparing a solid phase immunoassay, which process comprises:

purifying a specific binding protein;

reacting the purified specific binding protein with one or more aldehyde to enhance the hydrophobic nature of the protein without crosslinking or polymerizing the protein;

contacting the surface of an insoluble support with the enhanced hydrophobic specific binding protein in the presence of a buffer and bonding the specific binding protein to the surface; and

removing any remaining unbound protein from the insoluble support.

This method does not result in crosslinking the starting binding protein or increasing the molecular weight of the starting binding protein, and enhanced binding of high titre antiserum is achieved.

According to the second aspect of the present invention, there is provided a method of assaying a specimen for a specific component of the specimen, which method comprises:

reacting together, a sample of the specimen containing the specific unlabelled component and a specific labelled component equivalent to the unlabelled component, in the presence of a buffer, with a specific binding protein hydrophobically bound to an insoluble support, which specific binding protein is both complementary to the specific component and prepared by a process according to the first aspect of the present invention;

incubating the reactants;

decanting the unbound reactants; and

measuring the bound or unbound reactants and comparing the measurement with a standard curve.

According to a third aspect of the present invention, there is provided an immunoassay kit for assaying a specimen for a specific component of the specimen, which kit comprises:

an insoluble support, part of or all the surface being coated with an enhanced hydrophobic specific binding protein prepared by a process according to the first aspect of the present invention; and

a sample labelled component.

As used herein, the term "specific binding protein" means a group of serum proteins, also referred to as gamma globulins or immunoglobulins, that will specifically react with an antibody, antigen or hapten. Most of the specific binding proteins belong to the immunoglobulin subclass known as IgG, while other subclasses are referred to as IgA, IgM, IgD, and IgE. This term is also used to include certain binding proteins which recognize certain antigens, such as thyroxine binding globulin for thyroxine. The term "kit", as employed in the description, should generally be taken to mean a collection of all or some of the chemicals, including the assay tubes and instructions, necessary to do an immunoassay.

Preferably, the specific binding protein employed in all three aspects of the present invention is chosen from:

an antibody, preferably an antibody containing immunoglobulin, more preferably an antibody containing immunoglobulin and beta-globulin;

an antigen; and

a hapten.

The specific bonding protein may be any antiserum that has been previously purified

The specific binding protein is purified, preferably to enhance the specific immunoglobulin efficiency of the specific binding protein's active constituents when bonded to an insoluble support while concurrently decreasing the non-specific binding of the non-immunoglobulin fraction; thus, there can be an increase in specific immunoglobulin content of the specific binding protein (as bonded to the insoluble support) with a concurrent decrease in the content of non-specific non-immunoglobulin (as bonded). Any conventional purification procedure may be employed to purify the specific binding protein to remove non-essential fractions. Such procedures preferably result in the removal of serum albumin, i.e. α-1 and α-2 from the reamining mixed serum globulins. Removal of beta-globulin is not required and if removed may reduce the quantitative amount of specific binding protein present in the original sample.

Representative purification procedures include precipitation of the immunoglobulins with ammonium sulphate by conventional precipitation methods (see for example U.S. patent No. 4069352) or removal of unwanted material using solubilising organic solvents such as ethoxyacridine. These latter techniques involve selective solubilisation of the specific binding protein and precipitation of the remaining non-essential fractions. Once recovered, the purified specific binding protein is used or stored until ready for use.

The specific binding protein, once purified, is reacted with one or more aldehyde. It has been unexpectedly found that soluble specific binding protein can be reacted with an aldehyde to enhance the binding affinity of the protein for an insoluble support without crosslinking the protein, polymerizing the protein or increasing

the molecular weight of the protein.

While the exact mechanism for this effect is not known, it is believed to result from the carbonyl addition and/or acid-catalyzed carbonyl addition reactions of the respective specific binding protein to the aldehyde. These reactions may be depicted by the following representations wherein "Z" is the available amino groups on the specific binding protein and R is hydrogen, an alkyl group or an aryl group.

Carbonyl addition

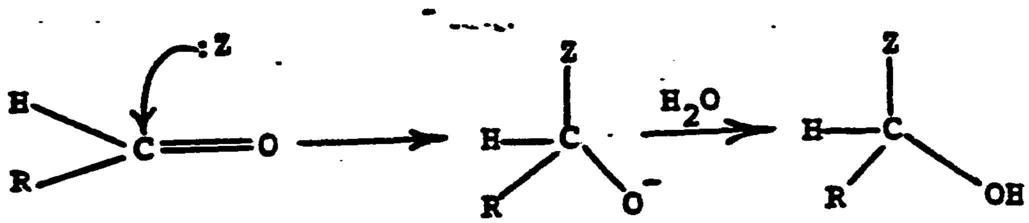


**Acid-catalyzed carbonyl addition**

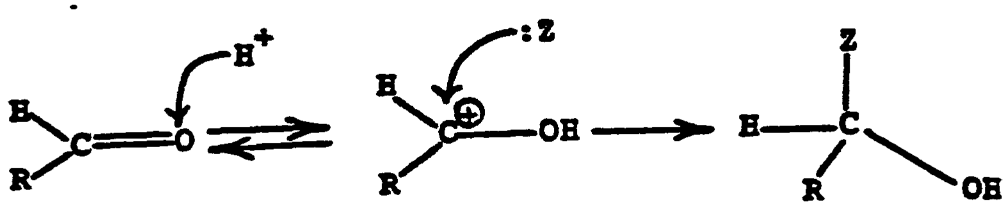

The noted reactions can occur over a narrow range of acidic and basic conditions. In order to ensure optimum reaction, however, use of a buffer to control pH during the reaction has been found preferable. Acceptable material is obtained when buffer is employed in suitable amounts to control the pH in the range from 6.0 to 7.8 and preferably in the range from 7.0 to 7.5. Values of pH above 7.8 are not is used to avoid denaturation of the protein, whereas pH values below 6.0 may result in acid hydrolysis of the protein. Any suitable buffer, which

does not affect the protein properties, may be selected for this purpose. Illustrative buffers include sodium carbonate, potassium carbonate, sodium phosphate (monobasic acid/or dibasic) potassium phosphate (monobasic- and/or dibasic), sodium chloride in a suitable buffer base and potassium chloride in a suitable buffer, and mixtures thereof. Once the components are mixed they are allowed to react, at temperatures below approximately 37°C, to form the aldehyde-protein conjugate. Temperatures should be maintained in the range from 4°C to 37°C and preferably in the range from 20°C to 30°C for optimum efficiency. The reaction time may vary greatly from a few minutes to several hours and is preferably in the range from 5 to 60 minutes.

The one or more aldehyde used in all three aspects of the present invention may be selected from a wide variety of monofunctional and bifunctional reagents which are water-soluble. Exemplary water-soluble monofunctional aldehydes may be represented by the formula R-CHO wherein R is a hydrogen atom, an alkyl group, or an aryl group. Representative aldehydes include formaldehyde, acetaldehyde, propionaldehyde, n-butyraldehyde and benzaldehyde, and mixtures thereof, with formaldehyde being preferred.

Exemplary water-soluble bifunctional material may be represented by the formula $OHC-R^1CHO$, wherein $R^1$ may be; an alkylene group, for example, butylene; a monocyclic alkylene group, for example, cyclopentylene or cyclohexylene; a monocyclic arylene group, for example, phenylene; a bicyclic arylene group, for example, naphthylene; a substituted monocyclic alkylene group; or a substituted monocyclic arylene group, for example, tolylene

and mixtures thereof. The preferred bifunctional aldehyde is glutaraldehyde.

The reaction product is an aldehyde-specific binding protein which does not exhibit crosslinking of the protein, polymerization of the protein or protein having increased molecular weight.

The aldehyde-specific binding protein obtained is then brought into contact with an insoluble support and permitted to bond to the internal surface of the support. This reaction can be obtained by incubation of the reaction product in the presence of the insoluble support under suitable temperature in the essential presence of a buffered solution. The temperature may vary widely depending upon the reaction time and coating efficiency desired and is preferably performed at temperatures in the range from 4°C to 30°C. Buffering is essential to achieve maximum coating efficiency and is performed at pH values below 8.0, preferably in the range from 6.0 to 7.5, more preferably in the range from 7.0 to 7.5. The pH value should be maintained during the entire coating period. Illustrative buffers include sodium carbonate, potassium carbonate, sodium phosphate (monobasic and dibasic), potassium phosphate (monobasic and dibasic), sodium chloride in a suitable buffer and potassium chloride in a suitable buffer, and mixtures thereof. The incubation time may vary widely depending on the degree of binding desired. Times in the range from 3 hours to 24 hours, preferably in the range from 10 hours to 20 hours have been found suitable to achieve bindings greater than 40% of the total amount of the protein.

The aldehyde treated specific bonding protein exhibits enhanced coating efficiency under the

reaction conditions described. The reason for this effect is not fully known but is believed to be caused by an increased hydrophobicity of the protein resulting from the aldehyde addition. This increased hydrophobicity is believed to result in increasing the binding affinity of the protein molecule to the insoluble support while limiting desorption of the specific binding protein when the support is in use.

Suitable solid support surfaces which may be employed in all three aspects of the present invention are formed from water-insoluble polymeric material capable of adsorbing the specific binding protein. Exemplary materials include hydrocarbon polymers such as polystyrene, polyethylene, polypropylene and polybutylene. Other suitable organic polymers include silastic rubber, polyesters, polyamides, cellulose and cellulosic derviatives, acrylates, methacrylates, and vinyl polymers such as vinyl chloride, and polyvinyl fluoride. Copolymers such as graft copolymers of polystryrene are also useful.

A particularly effective form for the solid support surface is a test-tube, bead, fin, well or microtitre plate. After completion of the contacting-adsorption reaction, unbound protein and solution are removed from the insoluble support and the support surface is allowed to dry. Drying may be achieved by conventional techniques such as air drying or oven drying overnight at ambient temperatures up to 50°C. The resulting immunoassay tube has long term stability and excellent reproducibility. Storage may be undertaken at suitable temperatures for example in the range 4°C to 30°C.

Because the solid phase immunoassay system prepared by this process demonstrates a high

degree of adsorption of specific binding protein without apparent loss or desorption when in use, this assay, in use, is a simple, rapid and practical means to provide an accurate analysis of small amounts of sample substances and is well suited for quantitative and qualitative determinations.

The method of the second aspect of the present invention provides a means of assaying a specimen for a specific component such as an antigen, hapten or antibody. A solid support surface is provided with a conjugate specific binding protein by the process of the first aspect of the present invention. The insoluble support is then treated by conventional procedures to determine the quantitative and/or qualitative amount of material to be analysed. For example, an unlabelled specimen and a labelled specific component equivalent to the unlabelled component may contact the surface of the solid support of this invention to permit competitive reaction. This is followed by separation of the bound and unbound material and measurement of the labelled component on the surface or in the removed solution to calculate the amount of specimen component originally present.

The labelled material used in the second and third aspects of the present invention may be any conventional label, such as a radioactive isotope, an enzyme or a fluorometric material. When the component is labelled with an enzyme as described, for example, in U.S. Patent No. 3791932, the amount of labelled component can be determined by measuring enzymatic activity of the tube. When the component is labelled with a fluorescent material the amount of labelled antigen can be determined by measuring fluorescense

as described in U.S. Patent No. 3789116. However the labelling is accomplished, the labelled material serves as a tracer.

A particularly preferred method involves labelling the specific binding protein with a radioactive isotope in a conventional manner. Suitable isotopes include $I^{125}$, $I^{131}$, $C^{14}$ or $H^3$. A particularly suitable isotope is the radioactive isotope of iodine, $I^{125}$, since labelling with this isotope is simple and many hospital laboratories have the equipment necessary to measure this material.

The immunoassay kit according to the third aspect of the present invention may containfor example, a sample labelled component chosen from: thyroxine ($T_4$), triodothyronine ($T_3$), digoxin, cortosol, theophylline, gentamycin, folic acid, and Vitamin $B_{12}$.

The present invention is further illustrated by the following examples. All parts and percentages in the examples as well as in the remainder of the description are by weight unless otherwise specified.

EXAMPLE 1

This example demonstrates a procedure for purifying an antiserum and its use in preparing a solid phase immunoassay according to the first aspect of the present invention.

Purification Procedure:

One millilitre rabbit antiserum specific for thyroxin ($T_4$) was mixed with four millilitres of a 0.4% solution of ethoxyacridine containing 0.001 molar sodium borate buffer. The buffered solution had a pH value of 8.0. The solution was mechanically mixed for 30 minutes at 24°C. The suspension was then centrifuged at 1500 to 2000 rpm for 15 minutes to separate undesired

insoluble matter, predominantly albumin, α -1 and α -2 globulins. The resulting supernatant solution was then dialyzed against a 0.01 molar isotonic phosphate buffer solution which had a pH value of 7.0. The dialyzed solution, which contained the specific rabbit antiserum binding protein which itself contained predominantly beta-globulin and immunoglobulin fractions, was then stored in glass vials at -20°C until used.

Adsorption Procedure:

A 0.25 millilitre aliquot of the purified rabbit antiserum was added to a buffer containing 0.01 molar phosphate buffer ($Na_2HPO_4$), 0.15 molar NaCl and 0.1 molar $NaN_3$ at a pH value of 6.0. To this solution was added glutaraldehyde to a final concentration of 0.005 molar. The resulting mixture was allowed to react for 20 minutes at 24°C and then mixed with 3 parts of a 0.7 molar phosphate buffer, pH 7.5. A 1.2 millilitre aliquot of the solution was added to dried polypropylene tubes and incubated for 18 hours at 24°C. The tubes were emptied of excess solution and washed twice with an isotonic 0.01 molar tris buffer, at pH 7.4, which contained 0.1% gelatin. The tubes were then air dried at 24°C and stored for use.

EXAMPLE 2

The procedure of Example 1 was repeated except that the adsorption reaction was conducted in a single stage with one buffer.

A 0.25 millilitre aliquot of the purified rabbit antiserum of Example 1 was added to various buffers at pH 7.5 as set forth in Table 1. To this solution was added glutaraldehyde to a final concentration of 0.005 molar. The resulting mixture was allowed to react for 20 minutes

at 24°C and 1.2 millilitres was added to dried polypropylene test tubes, incubated, and washed as in Exmaple 1.

A comparative analysis of tubes was made in the absence of glutaraldehyde as a reacting material.

The buffers employed and results obtained are described in Table 1. The tabulated results demonstrate % binding of the antiserum as calculated by competitive incubation of a constant fixed quantity of $T_4$ radioactively labelled with $I^{125}$ in the presence of known standard quantities of unlabelled $T_4$ as the diluent (represented by A, C and E in Tables 1). The values shown represent an average value of triplicate determinations of the number of counts in the bound fraction as a percentage of the total counts. A comparison of the average percent binding (Av % B/T) in the presence and absence of an aldehyde show increased coating efficiency by aldehyde activation.

The results indicate that passive adsorption is not as efficient in coating polypropylene tubes as is a glutaradlehyde treated antiserum according to this invention.

Table 1

| Buffer | Sample | Passive Av. & B/T | Glutar-aldehyde Av. % B/T |
|---|---|---|---|
| 0.5M $Na_2CO_3$ pH 7.5 | O µg/dl | 31.35 | 42.85 |
| | A 1.0 µg/dl | 28.31 | 39.43 |
| | C 11.7 µg/dl | 7.37 | 13.18 |
| | E 18.0 µg/dl | 5.16 | 8.71 |
| 0.0025M $Na_2HPO_4$ 0.5M NaCl pH 7.5 | O µg/dl | 28.30 | 43.93 |
| | A 1.0 µg/dl | 24.42 | 40.00 |
| | C 11.7 µg/dl | 6.98 | 13.64 |
| | E 18.0 µg/dl | 4.70 | 9.37 |
| 0.5M $Na_2HPO_4$ pH 7.5 | O µg/dl | 32.33 | 42.65 |
| | A 1.0 µg/dl | 24.42 | 40.01 |
| | C 11.7 µg/dl | 7.86 | 13.18 |
| | E 18.0 µg/dl | 5.50 | 8.60 |
| 0.5M $K_2HPO_4$ pH 7.5 | O µg/dl | 32.0 | 48.0 |
| | A 1.0 µg/dl | 27.0 | 42.0 |
| | C 11.7 µg/dl | 8.0 | 16.0 |
| | E 18.0 µg/dl | 6.0 | 11.5 |
| 0.5M KCl 0.0025M $K_2HPO_4$ pH 7.5 | O µg/dl | 30.0 | 48.0 |
| | A 1.0 µg/dl | 27.0 | 43.0 |
| | C 11.7 µg/dl | 8.5 | 15.5 |
| | E 18.0 µg/dl | 6.5 | 10.0 |

Note: Tracer
Total Counts 99.95%

EXAMPLE 3

The procedure of Example 1 was repeated except that formaldehyde was used in place of glutaraldehyde.

The results are set forth in Table 2 which shows the effect of using various amounts of formaldehyde. The results indicate improved coating efficiency with a monofunctional aldehyde, as compared to no aldehyde (passive). The abbreviations have the same meaning as in Table 1.

Table 2

| Formaldehyde concentration | Sample | Av. % B/T |
|---|---|---|
| 0.000M (Passive) | A 1.0 μg/dl | 36.72 |
| | C 11.7 μg/dl | 9.21 |
| 0.05M | A 1.0 μg/dl | 46.20 |
| | C 11.7 μg/dl | 13.29 |
| 0.075M | A 1.0 μg/dl | 47.80 |
| | C 11.7 μg/dl | 13.66 |
| 0.10M | A 1.0 μg/dl | 46.98 |
| | C 11.7 μg/dl | 13.70 |
| 0.25M | A 1.0 μg/dl | 46.30 |
| | C 11.7 μg/dl | 14.23 |

Note:

Tracer
Total Counts 99.79%

EXAMPLE 4

This example demonstrates the molecular weight characteristics of purified rabbit antiserum specific for $T_4$ prepared by Example 1 as compared to a range of molecular weight standards. The molecular weight standards used were computed from a kit obtained from Biorad Laboratores, California, U.S.A. The standard materials and their molecular weights were previously established as:

| Protein Standard | Molecular Weight (gram mole) |
|---|---|
| Void (protein aggregates) | >670,000 |
| Thyroxin binding protein | 670 000 |
| Gamma immunoglobulin | 158 000 |
| Ovalbumin | 44 000 |
| Myoglobulin | 17 000 |
| Vitamin-$B_{12}$ | 1 350 |

Figure 1 depicts a molecular weight profile using purified rabbit antiserum specific for $T_4$ without glutaraldehyde activation. Figure 2 depicts a molecular weight profile using purified rabbit antiserum specific for $T_4$ after glutaraldehyde activation by the method of the present invention.

The results clearly demonstrate no crosslinking or polymerization of the antiserum protein after reaction with glutaraldehyde.

EXAMPLE 5

This example demonstrates use of the solid phase immunoassay of this invention in the determination of a specific component.

To determine the quantitative amount of the specific component present in a sample specimen, 10 microlitres of the specimen was placed into a previously prepared antibody coated tube, which tube was prepared by the procedure of Example 1. Subsequently, a 1.0 millilitre sample of a radioactively labelled complementary antigen using $I^{125}$ in a 0.01 tris buffer pH 7.4, containing 0.015% anilinonaphthalene sulphonic acid was added to the antibody coated tube. The reactants were incubated for 60 minutes at 24°C. The reactants were decanted and the percent bound fraction, determined by a gamma counter which measures the $I^{125}$ activity, and the percent bound of the specific component,

were computed from a dose response curve using known standards.

The utility of the invention for accurate measurement of thyroxine in patients sera was demonstrated using sera having high, low and normal thyroxine levels. The results are tabulated below.

The patient Sample No. has no connection with the knwon standard quantities A, C and E of Tables 1 and 2.

| Patient Sample No. | | $T_4$ Assay µg/dl |
|---|---|---|
| (Hypo) | A | 4.8 |
| | B | 2.2 |
| | C | 2.0 |
| | D | 2.6 |
| | E | 4.5 |
| | F | 3.3 |
| | G | 3.9 |
| | H | 4.0 |
| | I | 4.2 |
| | J | 3.8 |
| (Norm) | AA | 7.4 |
| | BB | 6.2 |
| | CC | 7.6 |
| | DD | 8.0 |
| | EE | 7.3 |
| | FF | 9.1 |
| | GG | 10.3 |
| | HH | 10.3 |
| | II | 10.3 |
| | JJ | 9.8 |
| (Hyper) | AAA | 12.8 |
| | BBB | 15.0 |
| | CCC | 15.0 |
| | DDD | 13.6 |
| | EEE | 14.1 |
| | FFF | 16.2 |

| Patient Sample No. | | $T_4$ Assay µg/dl |
|---|---|---|
| (Hyper) | GGG | 15.9 |
| | HHH | 16.5 |
| | III | 18.3 |
| | JJJ | 19.1 |

CLAIMS:

1. A process for preparing a solid phase immunoassay, which process comprises:

purifying a specific binding protein;

reacting the purified specific binding protein with one or more aldehyde to enhance the hydrophobic nature of the protein without crosslinking or polymerizing the protein;

contacting the surface of an insoluble support with the enhanced hydrophobic specific binding protein in the presence of a buffer and bonding the specific binding protein to the surface; and

removing any remaining unbound protein from the insoluble support.

2. A process according to Claim 1, wherein the specific binding protein is chosen from:

an antibody, preferably an antibody containing immunoglobulin, more preferably an antibody containing immunoglobulin and beta-globulin;

an antigen; and

a hapten.

3. A process according to Claim 1 or 2, wherein the specific binding protein is a high titre antibody.

4. A process according to any preceding claim, wherein the one or more aldehyde is chosen from:

a monofunctional aldehyde, preferably having the formula R-CHO wherein R is a hydrogen atom, an alkyl group, or an aryl group, more preferably formaldehyde; and

a bifunctional aldehyde, preferably having the formula $OHC-R^1-CHO$ wherein $R^1$ is an alkylene group, a cycloalkylene group, a monocyclic arylene group, a bicyclic arylene

group, a substituted monocyclic alkylene group, or a substituted monocyclic arylene group, each of which has up to 10 carbon atoms, more preferably glutaraldehyde.

5. A process according to any preceding claim, wherein the reaction of the specific binding protein with the one or more aldehyde is performed in the presence of a buffer solution having a pH in the range from 6.0 to 7.8, preferably in the range 7.0 to 7.5.

6. A process according to any preceding claim, wherein the insoluble support is formed from a water-insoluble hydrophobic polymer.

7. A process according to any preceding claim, wherein the insoluble support is in the form of a test tube, a bead, a fin, a well, or a microtitre plate.

8. A process according to any preceding claim, wherein the enhanced hydrophobic specific binding protein is contacted with the insoluble support in the presence of a buffer having a pH below 8.0, preferably in the range from 6.0 to 7.5.

9. A process according to any preceding claim, wherein the buffer used during the contacting step, or a buffer used during the reaction between the specific binding protein and the one or more aldehyde, is chosen from: sodium carbonate, potassium carbonate, sodium phosphate, potassium phosphate, sodium chloride, potassium chloride, and mixtures thereof.

10. A method of assaying a specimen for a specific component of the specimen, which method comprises:

reacting together, a sample of the specimen containing the specific unlabelled component and a specific labelled component

equivalent to the unlabelled component, in the presence of a buffer, with a specific binding protein hydrophobically bound to an insoluble support, which specific binding protein is both complementary to the specific component and prepared by a process according to any preceding claim;

incubating the reactants;

decanting the unbound reactants; and

measuring the bound or unbound reactants and comparing the measurement with a standard curve.

11. A method according to Claim 10, wherein the specific labelled component is radioactively labelled, enzyme labelled or fluorescently labelled.

12. An immunoassay kit for assaying a specimen for a specific component of the specimen, which kit comprises:

an insoluble support, part of or all the surface being coated with an enhanced hydrophobic specific binding protein prepared by a process according to any one of Claims 1 to 9; and

a sample labelled component.

13. An immunoassay kit according to Claim 12, which contains a sample labelled component chosen from: thyroxine ($T_4$), triodothyronine ($T_3$), digoxin, cortosol, theophylline, gentamycin, folic acid, and Vitamin $B_{12}$.

FIG.1

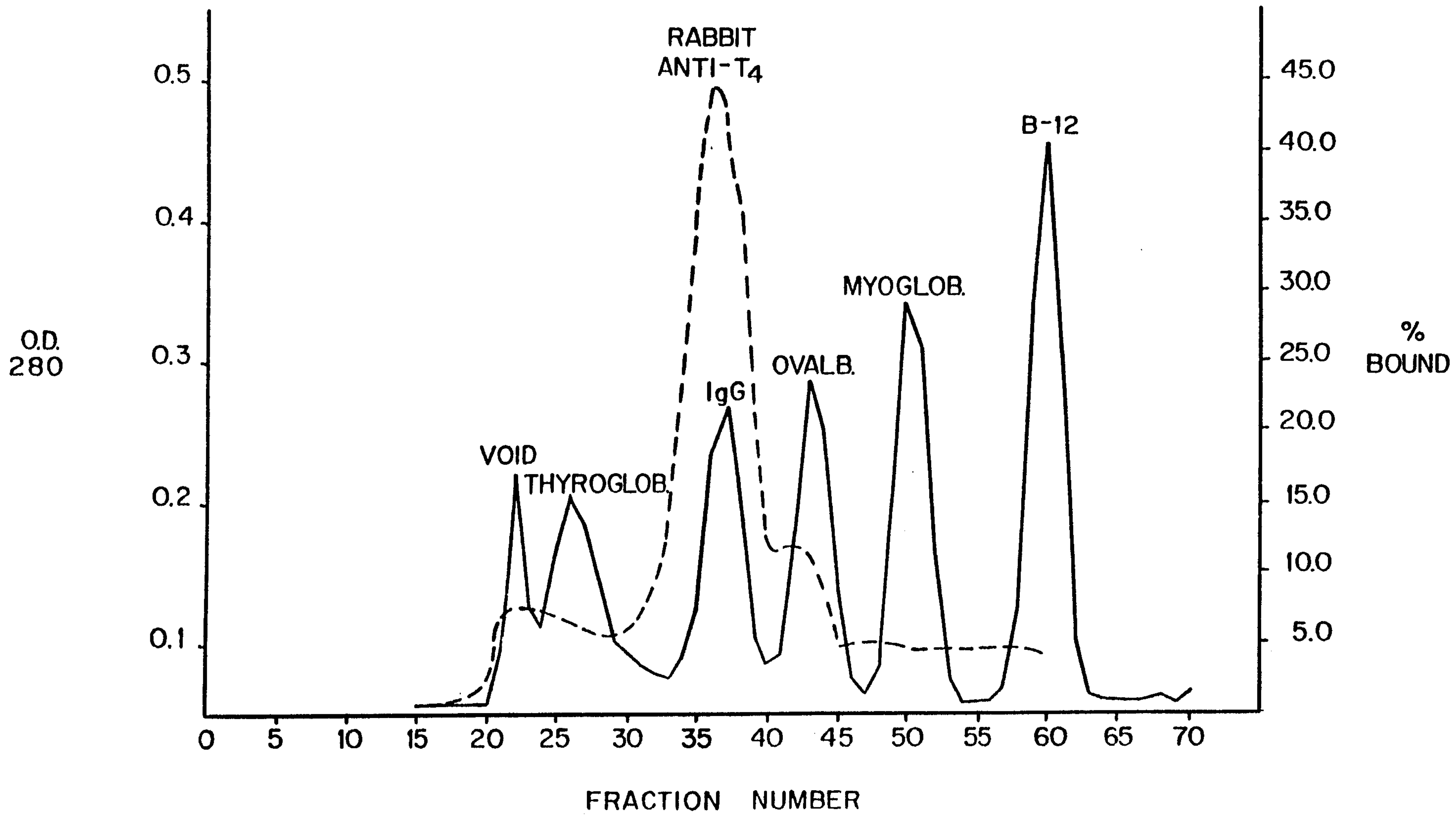
O.D. 280
0.5
0.4
0.3
0.2
0.1
RABBIT ANTI-$T_4$
B-12
MYOGLOB.
OVALB.
IgG
VOID
THYROGLOB.
% BOUND
45.0
40.0
35.0
30.0
25.0
20.0
15.0
10.0
5.0
0
5
10
15
20
25
30
35
40
45
50
55
60
65
70
FRACTION NUMBER

# FIG. 2

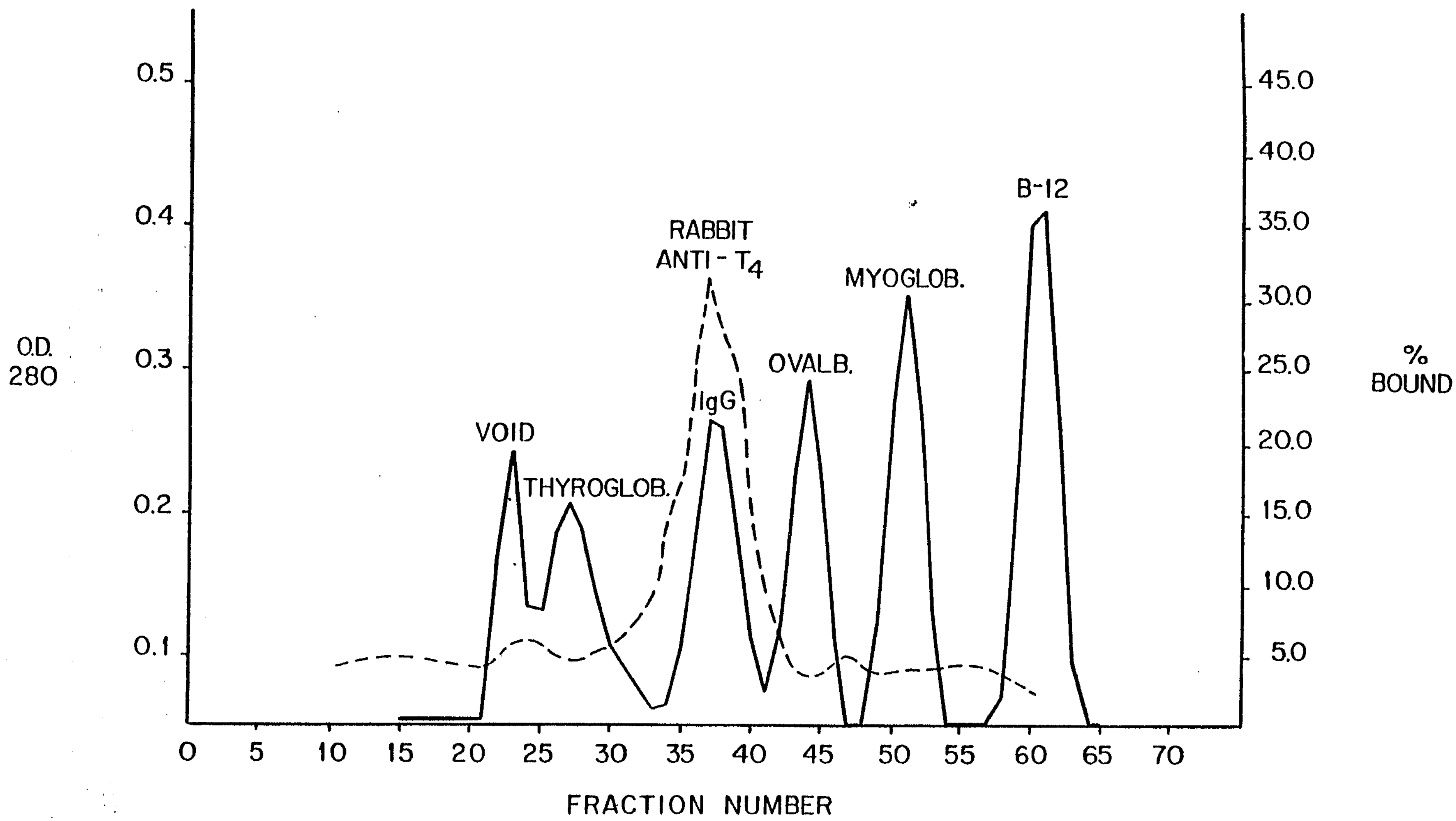
O.D.
280
0.5
0.4
0.3
0.2
0.1
% BOUND
45.0
40.0
35.0
30.0
25.0
20.0
15.0
10.0
5.0
VOID
THYROGLOB.
RABBIT ANTI - $T_4$
IgG
OVALB.
MYOGLOB.
B-12
0
5
10
15
20
25
30
35
40
45
50
55
60
65
70
FRACTION NUMBER

European Patent Office

# EUROPEAN SEARCH REPORT

0115681 Application number

EP 83307533.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | US - A - 4 069 352 (PARSONS JR.) * Abstract * -- | 1,11 | G 01 N 33/54 G 01 N 33/56 |
| D,A | US - A - 4 001 583 (BARRETT) * Abstract * -- | 1,3 | |
| D,A | US - A - 3 646 346 (CATT) * Claim 1 * -- | 1 | |
| A | US - A - 4 279 885 (REESE et al.) * Abstract * -- | 1 | |
| A | US - A - 4 244 694 (FARINA et al.) * Abstract * ---- | 1 | |

TECHNICAL FIELDS SEARCHED (Int. Cl. 3)

G 01 N 33/00

The present search report has been drawn up for all claims

| Place of search | Date of completion of the search | Examiner |
|---|---|---|
| VIENNA | 09-04-1984 | SCHNASS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82